# EUROPEAN PATENT SPECIFICATION

(11) **EP 4 215 109 B1**
(45) Date of publication and mention of the grant of the patent: **27.11.2024**
(21) Application number: 23151733.5
(22) Date of filing: 16.01.2023
(51) Int. Cl.: A61B 5/024, A61B 5/026, A61B 5/0533, A61B 5/11, A61B 5/369, A61B 5/00

(54) **APPARATUS AND COMPUTER-IMPLEMENTED METHOD FOR PROVIDING INFORMATION ABOUT A USER'S BRAIN RESOURCES, NON-TRANSITORY MACHINE-READABLE MEDIUM AND PROGRAM**
VORRICHTUNG UND COMPUTERIMPLEMENTIERTES VERFAHREN ZUR BEREITSTELLUNG VON INFORMATIONEN ÜBER GEHIRNRESSOURCEN EINES BENUTZERS, NICHTTRANSITORISCHES MASCHINENLESBARES MEDIUM UND PROGRAMM
APPAREIL ET PROCÉDÉ MIS EN UVRE PAR ORDINATEUR POUR FOURNIR DES INFORMATIONS CONCERNANT DES RESSOURCES CÉRÉBRALES D'UN UTILISATEUR, SUPPORT LISIBLE PAR MACHINE NON TRANSITOIRE ET PROGRAMME

(30) Priority: 21.01.2022 EP 22152667
(43) Date of publication of application: 26.07.2023
(73) Proprietor: Sony Group Corporation, Tokyo 108-0075 (JP)
(72) Inventor: ERYILMAZ, Serkan, 70327 Stuttgart (DE); JANSSENS, Inge, 70327 Stuttgart (DE); PFLUG, Anja, 70327 Stuttgart (DE); EMBRECHTS, Hugo, 70327 Stuttgart (DE); BAILADOR DEL POZO, Gonzalo, 70327 Stuttgart (DE)
(74) Representative: 2SPL Patentanwälte PartG mbB

(56) References cited:
- VAIBHAV JOSHI ET AL: "A Deep Knowledge Distillation framework for EEG assisted enhancement of single-lead ECG based sleep staging", ARXIV.ORG, CORNELL UNIVERSITY LIBRARY, 201 OLIN LIBRARY CORNELL UNIVERSITY ITHACA, NY 14853, 14 December 2021 (2021-12-14), XP091119730
- YANG G ET AL: "A driver fatigue recognition model based on information fusion and dynamic Bayesian network", INFORMATION SCIENCES, ELSEVIER, AMSTERDAM, NL, vol. 180, no. 10, 15 May 2010 (2010-05-15), pages 1942 - 1954, XP026937931, ISSN: 0020-0255, [retrieved on 20100114], DOI: 10.1016/J.INS.2010.01.011

## Description

### Field

The present disclosure relates to informing users about their brain resources. In particular, examples relate to an apparatus and a computer-implemented method for providing information about a user's brain resources, a non-transitory machine-readable medium and a program.

### Background

A user is confronted with a variety of tasks every day. However, the available brain energy of a user is limited. For example, it may be of interest for users to know the best time to do activities that require focus (e.g. a learning exercise or a difficult work assignment). Users subject to overcommitting may want to make sure they stay healthy. Other users may want to understand how to increase their brain resources.

Document Vaibhav Joshi et al.: "A Deep Knowledge Distillation framework for EEG assisted enhancement of single-lead ECG based sleep staging", ARXIV.ORG, CORNELL UNIVERSITY LIBRARY, 201 OLIN LIBRARY CORNELL UNIVERSITY ITHACA, NY 14853, 14 December 2021 (2021-12-14), XP091119730 proposes a cross-modal Knowledge Distillation (KD) framework to improve ElectroCardioGraphy (ECG)-based sleep staging performance with assistance from features learned through models trained on ElectroEncephaloGraphy (EEG).

There may be a demand for providing information about a user's brain resources.

### Summary

This demand is met by apparatuses and methods in accordance with the independent claims. Advantageous embodiments are addressed by the dependent claims.

According to a first aspect, the present disclosure provides an apparatus for providing information about a user's brain resources. The apparatus comprises a user interface, at least sensor interface circuitry and processing circuitry coupled to the sensor interface circuitry. In a calibration mode, the sensor interface circuitry is configured to receive first sensor data from an EEG sensor. The first sensor data are indicative of an electroencephalogram of the user. Further, the sensor interface circuitry is configured to receive second sensor data from a physiological sensor in the calibration mode. The second sensor data are indicative of a physiological property of the user. In the calibration mode, the processing circuitry is configured to train a brain-physiological model for the user based on the first sensor data and the second sensor data. The sensor interface circuitry is further configured to receive the second sensor data in an operation mode. In the operation mode, the processing circuitry is configured to determine the information about the user's brain resources by processing the second sensor data with the trained brain-physiological model. For determining the information about the user's brain resources, the processing circuitry is configured to not process data of the EEG sensor with the trained brain-physiological model. In the operation mode, the user interface is further configured to output a questionnaire regarding a physiological and/or cognitive status of the user as subjectively perceived by the user, and receive a user feedback to the questionnaire. The processing circuitry is in the operation mode further configured to train the brain-physiological model based on the user feedback to the questionnaire, determine a divergence between the user feedback to the questionnaire and the determined information about the user's brain resources, and, if the divergence is above a threshold level, initiate a re-calibration process for the brain-physiological model.

According to a second aspect, the present disclosure provides a computer-implemented method for providing information about a user's brain resources. The method comprises receiving, in a calibration mode, first sensor data from an EEG sensor at sensor interface circuitry. The first sensor data are indicative of an electroencephalogram of the user. Further, the method comprises receiving, in the calibration mode and an operation mode, second sensor data from a physiological sensor at the sensor interface circuitry. The second sensor data are indicative of a physiological property of the user. In addition, the method comprises training, in the calibration mode, a brain-physiological model for the user based on the first sensor data and the second sensor data. The method further comprises determining, in the operation mode, the information about the user's brain resources by processing the second sensor data with the trained brain-physiological model. For determining the information about the user's brain resources, data of the EEG sensor are not processed with the trained brain-physiological model. The method comprises outputting, in the operation mode, via a user interface a questionnaire regarding a physiological and/or cognitive status of the user as subjectively perceived by the user. In addition, the method comprises receiving, in the operation mode, via the user interface a user feedback to the questionnaire. Further, the method comprises training, in the operation mode, the brain-physiological model based on the user feedback to the questionnaire. In the operation mode, the method additionally comprises determining a divergence between the user feedback to the questionnaire and the determined information about the user's brain resources. If the divergence is above a threshold level, the method comprises initiating a re-calibration process for the brain-physiological model.

According to a third aspect, the present disclosure provides a non-transitory machine-readable medium having stored thereon a program having a program code for performing the above method for providing information about a user's brain resources, when the program is executed on a processor or a programmable hardware.

According to a fourth aspect, the present disclosure provides a program having a program code for performing the above method for providing information about a user's brain resources, when the program is executed on a processor or a programmable hardware.

### Brief description of the Figures

Some examples of apparatuses and/or methods will be described in the following by way of example only, and with reference to the accompanying figures, in which
Fig. 1 illustrates an example of an apparatus for providing information about a user's brain resources;
Fig. 2 illustrates an exemplary data flow; and
Fig. 3 illustrates a flowchart of an example of a method for providing information about a user's brain resources.

### Detailed Description

Some examples are now described in more detail with reference to the enclosed figures. However, other possible examples are not limited to the features of these embodiments described in detail. Other examples may include modifications of the features as well as equivalents and alternatives to the features. Furthermore, the terminology used herein to describe certain examples should not be restrictive of further possible examples.

Throughout the description of the figures same or similar reference numerals refer to same or similar elements and/or features, which may be identical or implemented in a modified form while providing the same or a similar function. The thickness of lines, layers and/or areas in the figures may also be exaggerated for clarification.

When two elements A and B are combined using an "or", this is to be understood as disclosing all possible combinations, i.e. only A, only B as well as A and B, unless expressly defined otherwise in the individual case. As an alternative wording for the same combinations, "at least one of A and B" or "A and/or B" may be used. This applies equivalently to combinations of more than two elements.

If a singular form, such as "a", "an" and "the" is used and the use of only a single element is not defined as mandatory either explicitly or implicitly, further examples may also use several elements to implement the same function. If a function is described below as implemented using multiple elements, further examples may implement the same function using a single element or a single processing entity. It is further understood that the terms "include", "including", "comprise" and/or "comprising", when used, describe the presence of the specified features, integers, steps, operations, processes, elements, components and/or a group thereof, but do not exclude the presence or addition of one or more other features, integers, steps, operations, processes, elements, components and/or a group thereof.

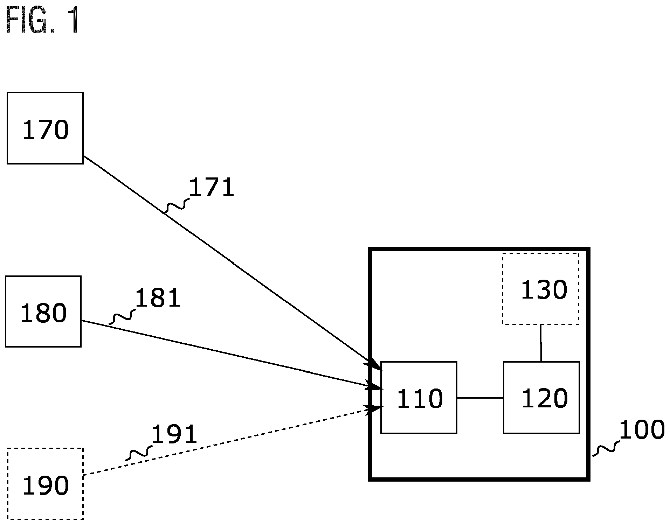

**Fig. 1** illustrates an exemplary apparatus 100 for providing information about a user's brain resources. The apparatus comprises at least sensor interface circuitry 110 and processing circuitry 120. For example, the processing circuitry 120 may be a single dedicated processor, a single shared processor, or a plurality of individual processors, some of which or all of which may be shared, a digital signal processor (DSP) hardware, an application specific integrated circuit (ASIC), a neuromorphic processor or a field programmable gate array (FPGA). The processing circuitry 120 is coupled to the sensor interface circuitry 110. The sensor interface circuitry 110 is for coupling the apparatus 100 to various sensors. The processing circuitry 120 may optionally be coupled to, e.g., read only memory (ROM) for storing software, random access memory (RAM) and/or non-volatile memory. Optionally, the apparatus 100 may comprise further circuitry.

The apparatus 100 may be operated in a plurality of modes. The plurality of modes comprise at least a calibration mode and an operation (prediction/determination) mode.

In the calibration mode, the sensor interface circuitry is configured to receive first sensor data 171 from an EEG sensor 170 and further second sensor data 181 from a physiological sensor 180. The first sensor data 171 are indicative of an electroencephalogram of the user. The second sensor data 181 are indicative of at least one physiological property (quantity, characteristic) of the user.

The physiological sensor 180 is configured to measure the physiological property (quantity, characteristic) of the user. The physiological property of the user is a property (quantity, characteristic) describing the physiology of the user. In other words, the physiological property is a property describing one or more function and/or mechanism in the user's body. For example, the physiological property may be one or more of a stress level of the user, a heart (pulse) rate of the user, a heart rate variability of the user, a cardiac cycle of the user, a respiration of the user, a blood pressure of the user, etc.

The physiological sensor 180 may be or be configured to perform the functionalities of one or more of a Laser Doppler Flowmetry (LDF) sensor, a PhotoPlethysmoGraphy (PPG) sensor, an EECG sensor or a Galvanic Skin Response (GSR) sensor. Accordingly, the second sensor data 181 may be sensor data of a LDF sensor, a PPG sensor, an ECG sensor and/or a GSR sensor.

An LDF sensor allows to detect blood volume changes in the microvascular bed of the user's tissue. Similarly, a PPG sensor allows to detect blood volume changes in the microvascular bed of the user's tissue. Accordingly, the second sensor data 181 may indicate measurement values of the blood volume changes measured by the PPG sensor or the LDF sensor. The measurement values of the blood volume changes measured by the PPG sensor or the LDF sensor are, e.g., indicative of a heart rate of the user, a heart rate variability of the user (e.g. ratio of Low Frequency power (LF) to High Frequency power (HF), also known as LF/HF ratio), a cardiac cycle of the user, a respiration of the user, a blood pressure of the user or a status of the autonomous nervous system of the user (e.g. stress or strong emotions).

A GSR sensor allows to measure the electrical conductance of the user's skin. Accordingly, the second sensor data 181 may indicate measurement values of the GSR measured by the GSR sensor. The measurement values of the GSR measured by the GSR sensor are, e.g., indicative of strong emotions and stress of the user. Strong emotions and stress can cause stimulus to the sympathetic nervous system of the user, resulting more sweat being secreted by the sweat glands of the user.

An ECG sensor allows to measure electrical signals generated by the heart of the user. Accordingly, the second sensor data 181 may indicate measurement values of the electrical signals measured by the ECG sensor. The measurement values of the electrical signals measured by the ECG sensor are, e.g., indicative of a heart rate of the user, a heart rate variability of the user, a cardiac cycle of the user, a physiological arousal or stress level that the user is experiencing.

The EEG sensor 170 is configured to measure the electroencephalogram of the user. The electroencephalogram is an electrogram of the electrical activity on the scalp of the user that represents the macroscopic activity of the surface layer of the user's brain underneath. The electroencephalogram is, hence, indicative of the user's brain activities.

The EEG sensor 170 and the physiological sensor 180 are illustrated as separate sensors in the example of Fig. 1. However, it is to be noted that the present disclosure is not limited thereto. In other examples, a single sensor may be configured to perform the functionalities of the EEG sensor 170 and the physiological sensor 180. The EEG sensor 170 and the physiological sensor 180 may be attached to the user's body at a respective suitable location (spot). For example, the EEG sensor 170 and the physiological sensor 180 may be integrated into one or more wearable (wearable computer) that can be worn on the user's body. For example, the physiological sensor 180 may be integrated into a (smart) wristband or a smartwatch to place the physiological sensor 180 near the wrist of the user such that the physiological sensor 180 may be worn conveniently by the user over long time periods. The EEG sensor 170 may, e.g., integrated into a (smart) headphone such that electrodes of the EEG sensor 170 may be placed near the skull of the user. Also the physiological sensor 180 may be integrated into a (smart) headphone or (smart) earbuds to place the physiological sensor 180 near an ear of the user such that the physiological sensor 180 may be worn conveniently by the user over long time periods.

The sensor interface circuitry 110 may receive the respective data 171, 181 directly from the EEG sensor 170 and the physiological sensor 180 or from communication circuitry of the apparatus 100, which is communicatively coupleable to the EEG sensor 170 and the physiological sensor 180. The apparatus 100 may be configured to wirelessly receive the respective data 171, 181 from the EEG sensor 170 and the physiological sensor 180. The sensor interface circuitry 110 or the communication circuitry of the apparatus 100 may be configured accordingly. The communication between the apparatus 100 and the respective one of the EEG sensor 170 and the physiological sensor 180 may be according to one of the 3rd Generation Partnership Project (3GPP)-standardized mobile/wireless communication networks or systems such as a 5^{th} Generation New Radio (5G NR) network, a Long-Term Evolution (LTE) network or an LTE-Advanced (LTE-A) network. Alternatively or additionally, communication between the apparatus 100 and the respective one of the EEG sensor 170 and the physiological sensor 180 may be according to mobile/wireless communication networks with different standards, for example, a Worldwide Inter-operability for Microwave Access (WIMAX) network according to the standard IEEE 802.16 of the Institute of Electrical and Electronics Engineers (IEEE), a Wireless Local Area Network (WLAN) according to the standard IEEE 802.11 of the IEEE, a Near-Field Communication (NFC) network, a Bluetooth network according to one of the standards of the Bluetooth Special Interest Group (SIG) or an Ultra-WideBand (UWB) network, generally an Orthogonal Frequency Division Multiple Access (OFDMA) network, a Time Division Multiple Access (TDMA) network, a Code Division Multiple Access (CDMA) network, a Wideband-CDMA (WCD-MA) network, a Frequency Division Multiple Access (FDMA) network, a Spatial Division Multiple Access (SDMA) network, etc.

The apparatus 100 may, e.g., be or be part of a mobile device such as a mobile phone (smartphone), a tablet-computer, a laptop-computer or a wearable device like a smart watch, smart glasses, etc. In still other examples, the apparatus 100 may, e.g., be or be part of a stationary device such as personal computer.

In the calibration mode, the processing circuitry 120 is configured to train a brain-physiological model for the user based on the first sensor data 171 and the second sensor data 181. The brain-physiological model for the user is a model of the user's brain-physiology. The brain-physiological model represents a maximum available brain energy (brain power/brain performance) of the user and user specific brain energy (power/performance) increase and decrease characteristics. In other words, the brain-physiological model models the brain energy and the brain energy changes of the user's brain. The trained brain-physiological model takes the second sensor data 181 as an input and outputs the information about the user's brain resources.

The sensor interface circuitry 110 is configured to receive the second sensor data 171 also in the operation mode. In the operation mode, the processing circuitry 120 is configured to determine the information about the user's brain resources by processing the second sensor data 171 with the trained brain-physiological model. For determining the information about the user's brain resources, the processing circuitry 120 is configured to not process data of the EEG sensor 170 with the trained brain-physiological model. In other words, the data of the EEG sensor 170 is only used for training (calibrating) the brain-physiological model, but not as input to the trained brain-physiological model for determining the information about the user's brain resources. That is, the processing circuitry 120 is configured to determine the information about the user's brain resources without processing data of the EEG sensor 170 with the trained brain-physiological model.

The information about the user's brain resources may indicate various properties (quantities, characteristics). The information about the user's brain resources may, e.g., indicate a respective estimated availably brain energy for one or more (future) daytime. For example, the information about the user's brain resources may indicate predicted brain energy levels throughout the (remaining) day, allowing the user to annotate activities (e.g. learning) to the predicted brain energy levels. According to some examples, the information about the user's brain resources may, e.g., indicate the remaining available brain energy through the day.

Alternatively or additionally, the information about the user's brain resources may, e.g., indicate one or more type of brain resources unconsciously consumed by the user. For example, focused or unfocused brain resources unconsciously consumed by the user throughout the day may be determined using the trained brain-physiological model. Further alternatively or additionally, the information about the user's brain resources may, e.g., indicate a history of brain resources usage such that a user can analyze the brain resources usage and better manage the available brain energy. Still further alternatively or additionally, the information about the user's brain resources may, e.g., indicate an expected point of time at which the user reaches a state of brain fatigue (mental fatigue). Brain fatigue is similar to physical tiredness, except it is the user's mind instead of the user's muscles. When the user is brain fatigue, the user is temporarily unable to maintain good (e.g. optimal) cognitive performance. Brain fatigue may, e.g., manifest as somnolence, lethargy, directed attention fatigue, or disengagement.

The apparatus 100 may allow to determine information about the user's brain resources using the data 181 of the physiological sensor 180. Accordingly, a user need not wear the EEG sensor 170 in the operation mode. As can be seen from the above examples, various types of information about the user's brain resources may be derived from the data 181 of the physiological sensor 180 using the trained brain-physiological model. Accordingly, the apparatus 100 may enable the user to better understand and/or optimize usage of the user's brain resources.

For example, the apparatus 100 may comprise a user interface 130. The user interface may, e.g., comprise one or more of a (e.g. touch sensitive) display, a loudspeaker and a microphone. In the operation mode, the user interface 130 may be configured to output the determined information about the user's brain resources to the user. The user interface 130 may, e.g., be configured to provide the user with a graphical description (representation) of the different types of brain resources consumed unconsciously during daily live. For example, the user interface 130 may visualize the available brain resources (e.g. focused vs unfocused) or visualize historical brain expenditures. Likewise, the information about the user's brain resources output by the user interface 130 may help the user understand the brain resource cost of the user's activities. For example, the user interface 130 may output an alert (e.g. a message or a sound) in case the user is subjecting himself to brain fatigue. The output information about the user's brain resources may allow the user to optimize his schedule and to improve planning of brain consuming activities. The user interface 130 may, e.g., output a calendar view with expected brain resources spent/recovered throughout the days. Similarly, the user interface 130 may, e.g., output to the user a notification (e.g. a message or a sound) linked to a particular brain state or brain energy level. Additionally or alternatively, user interface 130 may output annotations to daily activities to help further interpretations.

The determined information about the user's brain resources may be used for various use cases. For example, the determined information about the user's brain resources may allow the user to leverage his physiological data to choose the best time to do activities that require focus (e.g. a learning exercise or a difficult work assignment). In other examples, the proposed technology may allow the user to combine brain resources data (i.e. the determined information about the user's brain resources) and work scheduling to make sure he stays healthy in case the user is subject to overcommitting. Similarly, the determined information about the user's brain resources may allow the user to understand how to increase his brain resources.

For training the brain-physiological model in the calibration mode, the processing circuitry 120 may be configured to personalize a raw brain-physiological model based on the first sensor data 171. The raw brain-physiological model is a raw model not yet adapted (customized) to the specific brain-physiology of the user. For example, the raw brain-physiological model may be based on brain research and represent basic mechanisms for brain energy increase and decrease that can be adapted (customized) to the specific brain-physiology of the user.

As described above, the electroencephalogram represented by the first sensor data 171 of the EEG sensor 170 is indicative of user's brain activities. Hence, the first sensor data 171 allow to personalize the raw brain-physiological model. Accordingly, for personalizing the raw brain-physiological model, the processing circuitry 120 may be configured to determine, based on the first sensor data 171, the maximum available brain energy of the user and the user specific brain energy increase and decrease characteristics. In particular, the electroencephalogram represented by the first sensor data 171 may allow to calculate user specific brain energy expenditure parameters (rates), which may, e.g., be integrated to determine the maximum available brain energy (overall brain energy) of the user. Likewise, the electroencephalogram represented by the first sensor data 171 may allow to calculate user specific brain energy gain parameters (rates), such that together with the energy expenditure parameters (rates) the user specific brain energy increase and decrease characteristics may be determined.

As described above, the data of the EEG sensor 170 is only used for training the brain-physiological model, but not as input to the trained brain-physiological model for determining the information about the user's brain resources. Accordingly, a user need not wear the EEG sensor 170 in the operation mode. For training the brain-physiological model in the calibration mode, the processing circuitry 120 is configured to determine a relation between the first sensor data 171 of the EEG sensor and the second sensor data 181 of the physiological sensor 180. In other words, the processing circuitry 120 is configured to determine a mapping between one or more value and/or change in the first sensor data 171 and one or more value and/or change in the second sensor data 181. As the first sensor data 171 is characteristic of the user's brain activities, the determination of the relation between the first sensor data 171 and the second sensor data 181 allows to determine a relation between the user's brain activities and the second sensor data 181. Therefore, for training the brain-physiological model in the calibration mode, the processing circuitry 120 is further configured to determine a relation between the second sensor data 181 and the specific brain energy increase and decrease characteristics based on the relation between the first sensor data 171 and the second sensor data 181. Accordingly, the trained brain-physiological model is able to determine brain energy increases and decreases based on the second sensor data 181 in the operation mode - without the need for further data of the EEG sensor 170. In other words, the data of the EEG sensor 170 are used for modelling the individual physiological peculiarities of the user in order to enhance the quality of the signals collected using the physiological sensor 180 (e.g. a LDF sensor).

Even though the data of the EEG sensor 170 is not used as input to the trained brain-physiological model for determining the information about the user's brain resources in operation mode, it is to be noted that the sensor interface circuitry 110 may be configured receive the first sensor data 171 also during the operation mode according to some examples. For example, if the user wears the EEG sensor 170 also while the apparatus 100 is in the operation mode, the sensor interface circuitry 110 may receive the first sensor data 171 also during the operation mode. The first sensor data 171 may also be used in the operation mode. For example, if the first sensor data 171 is received by the sensor interface circuitry 110 while the apparatus 110 is in the operation mode, the processing circuitry 120 may, in the operation mode, be configured to further train the brain-physiological model based on the first sensor data 171 received while the apparatus 110 is in the operation mode. In other words, the already trained brain-physiological model may be further trained based on the first sensor data 171 received while the apparatus 110 is in the operation mode to further enhance (improve) the trained brain-physiological model on-the-fly. The additional training of the brain-physiological model in the operation mode may be done as described above for the training in the calibration mode.

The brain-physiological model may be trained based on further sensor inputs in the calibration. As illustrated in Fig. 1, the sensor interface circuitry 110 may, in the calibration mode, further be configured to receive contextual data 191 from at least one contextual sensor 190. The communication between the apparatus 100 and the at least one contextual sensor 190 may be as described above for the EEG sensor 170 and the physiological sensor 180. The at least one contextual sensor 190 may, e.g., be attached to the user's body or be worn by the user. The at least one contextual sensor 190 may be integrated into one or more wearable that can be worn on the user's body (e.g. a mobile phone or a smart watch). The contextual data 191 are indicative of an activity performed by the user (e.g. sports, sitting, walking, driving, eating, etc.). For example, the at least one contextual sensor 190 may be an acceleration sensor such that the contextual data 191 are indicative of a respective movement of one or more body part of the user. Additionally or alternatively, the at least one contextual sensor 190 may be a position sensor (e.g. using a global navigation satellite system such as GPS, GLONASS, Galileo or Beidou) such that the contextual data 191 are indicative of a position or position change of the user. The information about the activity performed by the user may allow to improve the interpretation of the electroencephalogram represented by the first sensor data 171 of the EEG sensor 170. Therefore, the processing circuitry 120 may in some examples be configured to train the brain-physiological model further based on the contextual data 191 in the calibration mode. For training the brain-physiological model in the calibration mode, the processing circuitry 120 may configured to determine a relation between the first sensor data 171 of the EEG sensor and the contextual data 191. In other words, the processing circuitry 120 may be configured to determine a mapping between one or more value and/or change in the first sensor data 171 and one or more value and/or change in the contextual data 191. As the first sensor data 171 is characteristic of the user's brain activities, the determination of the relation between the first sensor data 171 and the contextual data 191 allows to determine a relation between the user's brain activities and an activity performed by the user. The contextual data 191 may further be used to increase the quality of the readings of the EEG sensor 170 and/or the physiological sensor 180. For example, the processing circuitry 120 may be configured to use the contextual data 191 for reducing motion-related noise in the first sensor data 171 and/or the second sensor data 181.

The training of the brain-physiological model is not restricted to the usage of only sensor data. User experience is additionally used for the training of the brain-physiological model. For example, in the calibration mode, the user interface 130 may be configured to output a questionnaire regarding a physiological and/or cognitive status of the user as subjectively perceived by the user. Further, the user interface 130 may be configured to receive a user feedback to the questionnaire. For example, the user interface 130 may output the questionnaire via one or more graphical representation and/or one or more audio output. Similarly, the user interface 130 may receive the user feedback via one or more user input at a touch-sensitive display of the user interface 130 and/or one or more voice input of the user. The questionnaire may comprise various questions for querying the physiological and/or cognitive status of the user as subjectively perceived by the user. For example, the questionnaire may comprise questions like "Do you feel fatigue?", "Do you feel brain fatigue?" or "How motivated do you feel?". The processing circuitry may, in the calibration mode, accordingly be configured to train the brain-physiological model for the user further based on the user feedback to the questionnaire. The questionnaire allows the user to provide his subjective feeling and may, hence, allow to improve brain-physiological model. In particular, the data 171, 181 (and optionally the data 191) of the various sensors may be linked with the user's feedback to the questionnaire in order to train the brain-physiological model. For example, the training of brain-physiological model may comprise automatically computing a brain energy scale, containing the maximal available brain energy for the particular user, but also remaining available brain energy throughout the day. As described above, this scale is dynamic and changes according to time as well as activities that are done by the user (and sensed by the various sensors).

The brain-physiological model may be a machine-learning model. The machine-learning model is a data structure and/or set of rules representing a statistical model that the processing circuitry 120 uses to determine the information about a user's brain resources without using explicit instructions, instead relying on models and inference. The data structure and/or set of rules represents learned knowledge (e.g. based on training performed by a machine-learning algorithm as described above and below). Instead of a rule-based transformation of data, a transformation of data is used, that is inferred from an analysis of the various data in the calibration mode. As described above, the data 171, 181 and optionally at least one of the data 191 and the user feedback to the questionnaire is analyzed to train the machine-learning model (i.e. a data structure and/or set of rules representing the model). The trained machine-learning model is then able to determine the information about a user's brain resources by analyzing the data 181 of the physiological sensor 180 (and optionally further data).

The machine-learning model is trained by a machine-learning algorithm. The term "machine-learning algorithm" denotes a set of instructions that are used to create, train or use a machine-learning model. For the machine-learning model to analyze the content of the data 181 of the physiological sensor 180, the machine-learning model is trained in the calibration mode as described above. The data 171, 181 and optionally at least one of the data 191 and the user feedback to the questionnaire serve as training data. By training the machine-learning model with a large set of training data, the machine-learning model "learns" to recognize the content of the data 181 of the physiological sensor 180, so that the data 181 of the physiological sensor 180 that is not included in the training data can be recognized using the machine-learning model. By training the machine-learning model using training data, the machine-learning model "learns" a transformation between the data 181 of the physiological sensor 180 and the user's brain resources, which can be used to provide an output based on non-training data provided to the machine-learning model.

For example, unsupervised learning as described may be used to train the machine-learning model. In unsupervised learning, (only) input data are supplied and an unsupervised learning algorithm is used to find structure in the input data such as the data 171, 181 and optionally at least one of the data 191 and the user feedback to the questionnaire serve (e.g. by grouping or clustering the input data, finding commonalities in the data). Clustering is the assignment of input data comprising a plurality of input values into subsets (clusters) so that input values within the same cluster are similar according to one or more (pre-defined) similarity criteria, while being dissimilar to input values that are included in other clusters.

For example, the machine-learning model may be an Artificial Neural Network (ANN). ANNs are systems that are inspired by biological neural networks, such as can be found in a retina or a brain. ANNs comprise a plurality of interconnected nodes and a plurality of connections, so-called edges, between the nodes. There are usually three types of nodes, input nodes that receiving input values (e.g. the data 181 of the physiological sensor 180), hidden nodes that are (only) connected to other nodes, and output nodes that provide output values (e.g. user's brain resources). Each node may represent an artificial neuron. Each edge may transmit information from one node to another. The output of a node may be defined as a (non-linear) function of its inputs (e.g. of the sum of its inputs). The inputs of a node may be used in the function based on a "weight" of the edge or of the node that provides the input. The weight of nodes and/or of edges may be adjusted in the learning process. In other words, the training of an ANN may comprise adjusting the weights of the nodes and/or edges of the ANN, i.e., to achieve a desired output for a given input.

Alternatively, the machine-learning model may be a Generative Adversarial Network (GAN). In a GAN, two neutral networks contest with each other in a game (in the form of a zero-sum game, where one agent's gain is another agent's loss). Given a training set (such as the data 171, 181 and optionally at least one of the data 191 and the user feedback to the questionnaire), this technique learns to generate new data with the saint statistics as the training set. The basic principle of a GAN is based on the "indirect" training through a discriminator which itself is also being updated dynamically. This basically means that the generator is not trained to minimize the distance to a specific image, but rather to fool the discriminator. This enables the model to learn in an unsupervised manner.

Further alternatively, the machine-learning model may be a support vector machine, a random forest model or a gradient boosting model. Support vector machines (i.e. support vector networks) are supervised learning models with associated learning algorithms that may be used to analyze data (e.g. in classification or regression analysis). Support vector machines may be trained by providing an input with a plurality of training input values that belong to one of two categories (e.g. two different brain energy levels). The support vector machine may be trained to assign a new input value to one of the two categories. Alternatively, the machine-learning model may be a Bayesian network, which is a probabilistic directed acyclic graphical model. A Bayesian network may represent a set of random variables and their conditional dependencies using a directed acyclic graph. Alternatively, the machine-learning model may be based on a genetic algorithm, which is a search algorithm and heuristic technique that mimics the process of natural selection. In some examples, the machine-learning model may be a combination of the above examples.

The present disclosure is not limited to using questionnaires only in the calibration mode. Questionnaires are used in the operation mode as well. In the operation mode, the user interface 130 is configured to output a second questionnaire regarding the physiological and/or cognitive status of the user as subjectively perceived by the user. Accordingly, the user interface is configured to receive a second user feedback to the second questionnaire.

The second user feedback is used in various ways. The processing circuitry 120 is configured to train the brain-physiological model in the operation mode based on the second user feedback to the second questionnaire similar to what is described above for the questionnaire in the calibration mode. Accordingly, the brain-physiological model may be further improved while the apparatus 100 is in the operation mode.

The personalized brain-physiological model for the user is further improved by comparing one or more prediction of the brain-physiological model (i.e. the determined information about the about the user's brain resources) with the user's subjective experience (i.e. the second user feedback to the second questionnaire). When the predictions are too far from the user's personal experience, re-calibration of the brain-physiological model is performed. In the operation mode, the processing circuitry is configured to determine a divergence between the second user feedback to the second questionnaire and the determined information about the user's brain resources. If the divergence is above (larger than) a threshold level, the processing circuitry is configured to initiate a re-calibration process for the brain-physiological model.

The second questionnaire may be output to the user one or more time while the apparatus is in the operation mode. The second questionnaire may be output to the user at regular or irregular intervals. For example, in the operation mode, the user interface 130 may be configured to output the second questionnaire repeatedly throughout a day (e.g. two, three, four or more times during one day).

According to examples of the present disclosure, the contextual data 191 may also be used in the operation mode for determining the information about the user's brain resources. That is, the sensor interface circuitry 110 may also in the operation mode be configured to receive contextual data 191 from the at least one contextual sensor 190. Accordingly, the processing circuitry 120 may be configured to determine the information about the user's brain resources by processing the contextual data 191 in addition to the second sensor data 181 with the trained brain-physiological model. In other words, the trained brain-physiological model may take the second sensor data 181 and additionally the contextual data 191 as inputs in some examples of the present disclosure. Associating the second sensor data 181 with the contextual data 191 may allow improved interpretation of the input data and, hence, improve the determination of the information about the user's brain resources.

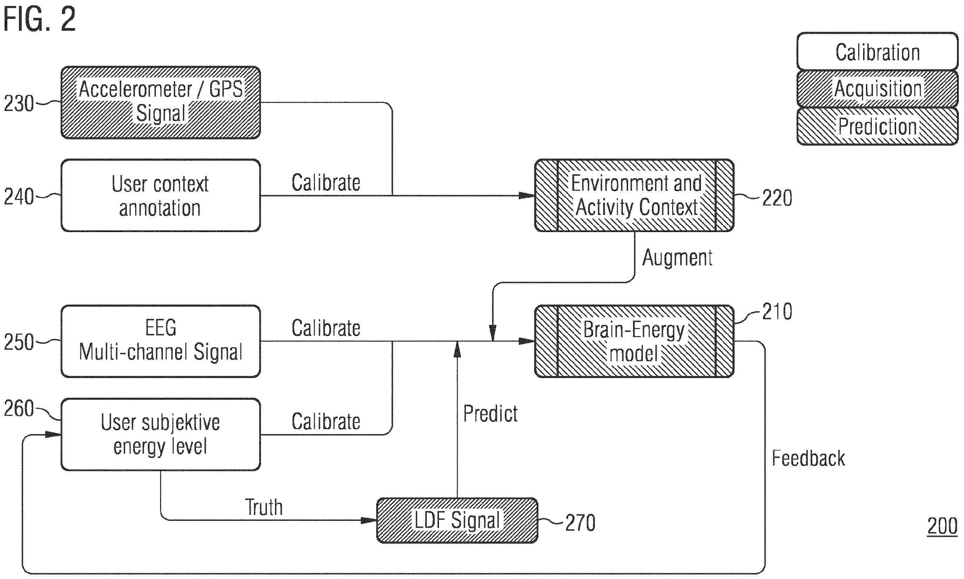

**Fig. 2** illustrates an exemplary data flow 200 according to the present disclosure. Fig. 2 illustrates a high-level overview of the working principle of an example of the present disclosure. A brain-physiological model 210 (which may also be understood as a brain-energy model) is used for determining/predicting the user's brain resources.

Different sensors worn by the user are providing data to an application in the calibration mode which then interprets this data and allows the user to provide his subjective feeling throughout the day. The application then creates the user adapted brain-physiological model 210 by linking the sensor data data with the users' input. In the example of the Fig. 2, the sensor data 250 of the EEG sensor and the sensor data 270 of the physiological sensor (e.g. a LDF sensor) together with the user annotated subjective energy level, i.e. the user feedback 260 to the questionnaire, is used in the calibration mode to build the brain-physiological model 210. In other words, the user wears all sensors in the calibration mode (phase/stage) such that the relationship between the sensor data 270 of the physiological sensor and the sensor data 250 of the EEG sensor can be calibrated in the calibration mode. Further, the contextual data 230 of the at least one contextual sensor (e.g. an acceleration sensor and/or a GPS sensor) may be received in the calibration mode such that also the relationship between the contextual data 230 of the at least one contextual sensor and the sensor data 250 of the EEG sensor can be calibrated in the calibration mode.

In the operation mode (phase/stage), which may also be understood as a data acquisition and prediction stage, the sensor data 250 of the EEG sensor are not mandatory. The calibrated brain-physiological model 210 is used to determine/predict various information about the user's brain resources using the sensor data 270 of the physiological sensor and optionally the contextual data 230 of the at least one contextual sensor. For example, the calibrated brain-physiological model 210 may allow to automatically compute a brain energy scale, containing the maximal available brain energy for the particular user, but also remaining available brain energy throughout the day. This scale is dynamic and changes according to time as well as activities that are done by the user (and indicated by the sensor data 270 of the physiological sensor and optionally the contextual data 230 of the at least one contextual sensor). For example, measurement data of a LDF signal may be used to transform blood-flow derived features to feed into the brain-physiological model 210 calibrated based on the sensor data 250 of the EEG sensor and the subjective experience 260 of the user.

The EEG sensor is used in the calibration mode to create the brain-physiological model 210 for the particular user. Then, the physiological sensor (e.g. a LDF sensor) is used as a proxy for the EEG sensor to infer mental energy levels that can be interpreted from physiological property measured by the physiological sensor (e.g. the blood flow alone during daily activities), even when the EEG sensor (e.g. included in headphones) is not worn.

Contextual information including the contextual data 230 of the at least one contextual sensor and optionally further user annotations 230 in the calibration mode may be used to augment the predictive power of the brain-physiological model 210. The contextual data (e.g. location, activity and/or time) may, e.g., be aggregated on a mobile device such as smartphone or a smartwatch of the user. The contextual information serves as an environment and activity context 220 for the brain-physiological model 210. As described above, this data may be used for the benefit of the user to understand how his brain energy varies within these contexts.

All the data gathered by various sensors may be strictly kept on the devices themselves and never sent to any external party, unless otherwise agreed by the user himself, and in return of clearly defined benefits that this would provide the user.

The feedback loop illustrated in Fig. 2 may allow to improve the model 210's predictive power if the user finds it incoherent with his subjective experience.

The application may, e.g., run on a smart phone and allow the user to interact with the proposed architecture for determining information about the user's brain resources. The user may, e.g., navigate the application using a touchscreen and be presented with different graphic and textual information about his brain activity. The brain activity may be presented to him only upon authentication. The application does not direct the user, however, it may compute correlations between activities and brain resources and, hence, allow the user to interpret his physiological data w.r.t his own subjective experience.

Accordingly, the application may allow the user to optimize the allocation of brain resources using data of various sensors.

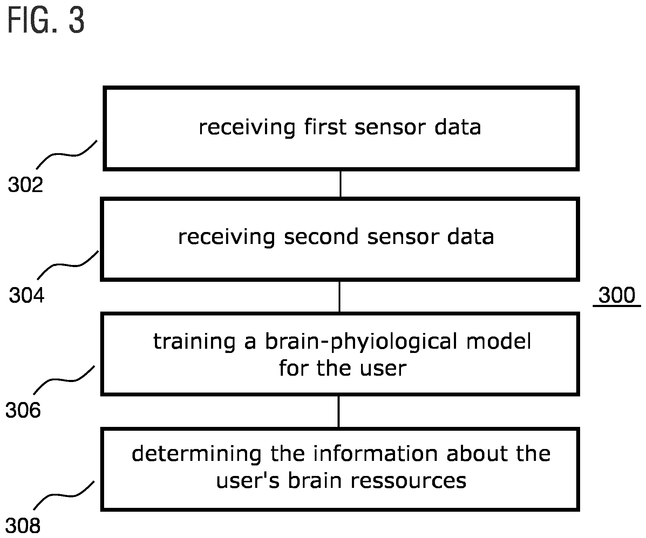

For further illustrating the proposed provision of information about a user's brain resources, **Fig. 3** illustrates a flowchart of a computer-implemented method 300 for providing information about a user's brain resources. The method 300 comprises receiving 302, in a calibration mode, first sensor data from an EEG sensor at sensor interface circuitry. The first sensor data are indicative of an electroencephalogram of the user. Further, the method 300 comprises receiving 304, in the calibration mode and an operation mode, second sensor data from a physiological sensor at the sensor interface circuitry. The second sensor data are indicative of a physiological property of the user. In addition, the method 300 comprises training 306, in the calibration mode, a brain-physiological model for the user based on the first sensor data and the second sensor data. The method 300 further comprises determining 308, in the operation mode, the information about the user's brain resources by processing the second sensor data with the trained brain-physiological model. For determining 308 the information about the user's brain resources, data of the EEG sensor are not processed with the trained brain-physiological model.

The method 300 may allow to determine information about the user's brain resources using the data of the physiological sensor. Accordingly, a user need not wear the EEG sensor in the operation mode. Further, the method 300 may enable the user to better understand and/or optimize usage of the user's brain resources.

More details and aspects of the method 300 are explained in connection with the proposed technique or one or more examples described above (e.g. Fig. 1 or Fig. 2). The method 300 may comprise one or more additional optional features corresponding to one or more aspects of the proposed technique or one or more examples described above.

Examples of the present disclosure may allow to optimize the allocation of brain resources using physiological sensors.

Examples may further be or relate to a (computer) program including a program code to execute one or more of the above methods when the program is executed on a computer, processor or other programmable hardware component. Thus, steps, operations or processes of different ones of the methods described above may also be executed by programmed computers, processors or other programmable hardware components. Examples may also cover program storage devices, such as digital data storage media, which are machine-, processor- or computer-readable and encode and/or contain machine-executable, processor-executable or computer-executable programs and instructions. Program storage devices may include or be digital storage devices, magnetic storage media such as magnetic disks and magnetic tapes, hard disk drives, or optically readable digital data storage media, for example. Other examples may also include computers, processors, control units, (field) programmable logic arrays ((F)PLAs), (field) programmable gate arrays ((F)PGAs), graphics processor units (GPU), application-specific integrated circuits (ASICs), integrated circuits (ICs) or system-on-a-chip (SoCs) systems programmed to execute the steps of the methods described above.

It is further understood that the disclosure of several steps, processes, operations or functions disclosed in the description or claims shall not be construed to imply that these operations are necessarily dependent on the order described, unless explicitly stated in the individual case or necessary for technical reasons. Therefore, the previous description does not limit the execution of several steps or functions to a certain order. Furthermore, in further examples, a single step, function, process or operation may include and/or be broken up into several sub-steps, -functions, -processes or -operations.

If some aspects have been described in relation to a device or system, these aspects should also be understood as a description of the corresponding method. For example, a block, device or functional aspect of the device or system may correspond to a feature, such as a method step, of the corresponding method. Accordingly, aspects described in relation to a method shall also be understood as a description of a corresponding block, a corresponding element, a property or a functional feature of a corresponding device or a corresponding system.

The following claims are hereby incorporated in the detailed description, wherein each claim may stand on its own as a separate example. It should also be noted that although in the claims a dependent claim refers to a particular combination with one or more other claims, other examples may also include a combination of the dependent claim with the subject matter of any other dependent or independent claim. Such combinations are hereby explicitly proposed, unless it is stated in the individual case that a particular combination is not intended. Furthermore, features of a claim should also be included for any other independent claim, even if that claim is not directly defined as dependent on that other independent claim.

## Claims

1. An apparatus (100) for providing information about a user's brain resources, the apparatus (100) comprising a user interface (130), at least sensor interface circuitry (110) and processing circuitry (120) coupled to the sensor interface circuitry (110),
wherein, in a calibration mode, the sensor interface circuitry (110) is configured to:
receive first sensor data (171) from an electroencephalography sensor (170), the first sensor data (171) being indicative of an electroencephalogram of the user; and
receive second sensor data (181) from a physiological sensor (180), the second sensor data (181) being indicative of a physiological property of the user,
wherein, in the calibration mode, the processing circuitry (120) is configured to train a brain-physiological model for the user based on the first sensor data (171) and the second sensor data (181),
wherein the sensor interface circuitry (110) is further configured to receive the second sensor data (181) in an operation mode,
wherein, in the operation mode, the processing circuitry (120) is configured to determine the information about the user's brain resources by processing the second sensor data (181) with the trained brain-physiological model,
wherein, for determining the information about the user's brain resources, the processing circuitry (120) is configured to not process data of the electroencephalography sensor with the trained brain-physiological model,
**characterized in that** in the operation mode: the user interface (130) is further configured to:
output a questionnaire regarding a physiological and/or cognitive status of the user as subjectively perceived by the user; and
receive a user feedback to the questionnaire, and
the processing circuitry (120) is further configured to:
train the brain-physiological model based on the user feedback to the questionnaire;
determine a divergence between the user feedback to the questionnaire and the determined information about the user's brain resources; and
if the divergence is above a threshold level, initiate a re-calibration process for the brain-physiological model.

2. The apparatus (100) of claim 1, wherein, for training the brain-physiological model, the processing circuitry (120) is configured to personalize a raw brain-physiological model based on the first sensor data (171).

3. The apparatus (100) of claim 2, wherein, for personalizing the raw brain-physiological model, the processing circuitry (120) is configured to determine, based on the first sensor data (171), a maximum available brain energy of the user and user specific brain energy increase and decrease characteristics.

4. The apparatus (100) of claim 3, wherein, for training the brain-physiological model, the processing circuitry (120) is configured to:
determine a relation between the first sensor data (171) and the second sensor data (181); and
determine a relation between the second sensor data (181) and the specific brain energy increase and decrease characteristics based on the relation between the first sensor data (171) and the second sensor data (181).

5. The apparatus (100) of any one of claims 1 to 4, wherein, in the calibration mode, the sensor interface circuitry (110) is further configured to receive contextual data (191) from at least one contextual sensor (190), the contextual data (191) being indicative of an activity performed by the user, and wherein the processing circuitry (120) is configured to train the brain-physiological model further based on the contextual data (191).

6. The apparatus (100) of claim 5, wherein the at least one contextual sensor (190) is an acceleration sensor, and wherein the contextual data (191) are indicative of a respective movement of one or more body part of the user.

7. The apparatus (100) of claim 5 or claim 6, wherein the at least one contextual sensor (190) is a position sensor, and wherein the contextual data (191) are indicative of a geolocation of the user.

8. The apparatus (100) of any one of claims 1 to 7, wherein the second sensor data (181) is sensor data of a laser Doppler flowmetry sensor, a photoplethysmography sensor, an electrocardiography sensor or a galvanic skin response sensor.

9. The apparatus (100) of any one of claims 1 to 8, wherein the brain-physiological model is a machine-learning model.

10. The apparatus of any one of claims 1 to 9, wherein in the calibration mode:
the user interface is configured to:
output a second questionnaire regarding the physiological and/or cognitive status of the user as subjectively perceived by the user; and
receive a second user feedback to the questionnaire, and
the processing circuitry is configured to train the brain-physiological model for the user further based on the second user feedback to the second questionnaire.

11. The apparatus (100) of any one of claims 1 to 10, wherein, in the operation mode, the user interface (130) is configured to output the second questionnaire repeatedly throughout a day.

12. The apparatus (100) of any one of claims 1 to 11, wherein, if the first sensor data (171) is received by the sensor interface circuitry (110) while the apparatus (100) is in the operation mode, the processing circuitry (120) is, in the operation mode, configured to train the brain-physiological model based on the first sensor data (171) received while the apparatus (100) is in the operation mode.

13. The apparatus (100) of any one of claims 1 to 12, wherein in the operation mode:
the sensor interface circuitry (110) is further configured to receive contextual data (191) from at least one contextual sensor (190), the contextual data (191) being indicative of an activity performed by the user; and
the processing circuitry (120) is configured to determine the information about the user's brain resources by processing the contextual data (191) in addition to the second sensor data (181) with the trained brain-physiological model.

14. A computer-implemented method (300) for providing information about a user's brain resources, the method (300) comprising:
receiving (302), in a calibration mode, first sensor data from an electroencephalography sensor at sensor interface circuitry, the first sensor data being indicative of an electroencephalogram of the user;
receiving (304), in the calibration mode and an operation mode, second sensor data from a physiological sensor at the sensor interface circuitry, the second sensor data being indicative of a physiological property of the user;
training (306), in the calibration mode, a brain-physiological model for the user based on the first sensor data and the second sensor data;
determining (308), in the operation mode, the information about the user's brain resources by processing the second sensor data with the trained brain-physiological model, wherein, for determining the information about the user's brain resources, data of the electroencephalography sensor are not processed with the trained brain-physiological model;
outputting, in the operation mode, via a user interface a questionnaire regarding a physiological and/or cognitive status of the user as subjectively perceived by the user;
receiving, in the operation mode, via the user interface a user feedback to the questionnaire;
training, in the operation mode, the brain-physiological model based on the user feedback to the questionnaire;
determining, in the operation mode, a divergence between the user feedback to the questionnaire and the determined information about the user's brain resources; and
if the divergence is above a threshold level, initiating a re-calibration process for the brain-physiological model.

15. A computer program having a program code for causing the apparatus of claim 1 to perform the method according to claim 14, when the program is executed on the processing circuitry of the apparatus of claim 1.

## Patentansprüche

1. Vorrichtung (100) zum Bereitstellen von Informationen über Gehirnressourcen eines Benutzers, die Vorrichtung (100) umfassend eine Benutzerschnittstelle (130), mindestens eine Sensorschnittstellenschaltung (110) und eine Verarbeitungsschaltung (120), die mit der Sensorschnittstellenschaltung (110) gekoppelt ist,
wobei, in einem Kalibrierungsmodus, die Sensorschnittstellenschaltung (110) konfiguriert ist zum:
Empfangen erster Sensordaten (171) von einem Elektroenzephalografiesensor (170), wobei die ersten Sensordaten (171) ein Elektroenzephalogramm des Benutzers anzeigen; und
Empfangen zweiter Sensordaten (181) von einem physiologischen Sensor (180), wobei die zweiten Sensordaten (181) eine physiologische Eigenschaft des Benutzers anzeigen,
wobei, in dem Kalibrierungsmodus, die Verarbeitungsschaltung (120) konfiguriert ist, um ein gehirnphysiologisches Modell für den Benutzer basierend auf den ersten Sensordaten (171) und den zweiten Sensordaten (181) zu trainieren,
wobei die Sensorschnittstellenschaltung (110) ferner konfiguriert ist, um die zweiten Sensordaten (181) in einem Betriebsmodus zu empfangen,
wobei, in dem Betriebsmodus, die Verarbeitungsschaltung (120) konfiguriert ist, um die Informationen über die Gehirnressourcen des Benutzers durch Verarbeiten der zweiten Sensordaten (181) mit dem trainierten gehirnphysiologischen Modell zu bestimmen,
wobei, zum Bestimmen der Informationen über die Gehirnressourcen des Benutzers, die Verarbeitungsschaltung (120) konfiguriert ist, um Daten des Elektroenzephalografiesensors mit dem trainierten gehirnphysiologischen Modell nicht zu verarbeiten,
**dadurch gekennzeichnet, dass** in dem Betriebsmodus:
die Benutzerschnittstelle (130) ferner konfiguriert ist zum:
Ausgeben eines Fragebogens bezüglich eines physiologischen und/oder kognitiven Zustands des Benutzers wie durch den Benutzer subjektiv wahrgenommen; und
Empfangen eines Benutzerfeedbacks zu dem Fragebogen, und
die Verarbeitungsschaltung (120) ferner konfiguriert ist zum:
Trainieren des gehirnphysiologischen Modells basierend auf dem Benutzerfeedback zu dem Fragebogen;
Bestimmen einer Abweichung zwischen dem Benutzerfeedback zu dem Fragebogen und den bestimmten Informationen über die Gehirnressourcen des Benutzers; und
falls die Abweichung über einem Schwellenwert liegt, Einleiten eines Neukalibrierungsprozesses für das gehirnphysiologische Modell.

2. Vorrichtung (100) nach Anspruch 1, wobei, zum Trainieren des gehirnphysiologischen Modells, die Verarbeitungsschaltung (120) konfiguriert ist, um ein rohes gehirnphysiologisches Modell basierend auf den ersten Sensordaten (171) zu personalisieren.

3. Vorrichtung (100) nach Anspruch 2, wobei, zum Personalisieren des rohen gehirnphysiologischen Modells,die Verarbeitungsschaltung (120) konfiguriert ist, um basierend auf den ersten Sensordaten (171) eine maximal verfügbare Gehirnenergie des Benutzers und benutzerspezifische Gehirnenergiezunahme-und -abnahmeeigenschaften zu bestimmen.

4. Vorrichtung (100) nach Anspruch 3, wobei, zum Trainieren des gehirnphysiologischen Modells, die Verarbeitungsschaltung (120) konfiguriert ist zum:
Bestimmen einer Beziehung zwischen den ersten Sensordaten (171) und den zweiten Sensordaten (181); und
Bestimmen einer Beziehung zwischen den zweiten Sensordaten (181) und den spezifischen Gehirnenergiezunahme- und -abnahmeeigenschaften basierend auf der Beziehung zwischen den ersten Sensordaten (171) und den zweiten Sensordaten (181).

5. Vorrichtung (100) nach einem der Ansprüche 1 bis 4, wobei, in dem Kalibrierungsmodus, die Sensorschnittstellenschaltung (110) ferner konfiguriert ist, um Kontextdaten (191) von mindestens einem Kontextsensor (190) zu empfangen, wobei die Kontextdaten (191) eine Aktivität anzeigen, die durch den Benutzer durchgeführt wird, und wobei die Verarbeitungsschaltung (120) konfiguriert ist, um das gehirnphysiologische Modell ferner basierend auf den Kontextdaten (191) zu trainieren.

6. Vorrichtung (100) nach Anspruch 5, wobei der mindestens eine Kontextsensor (190) ein Beschleunigungssensor ist und wobei die Kontextdaten (191) eine jeweilige Bewegung eines oder mehrerer Körperteile des Benutzers anzeigen.

7. Vorrichtung (100) nach Anspruch 5 oder 6, wobei der mindestens eine Kontextsensor (190) ein Positionssensor ist und wobei die Kontextdaten (191) eine Geolokalisierung des Benutzers anzeigen.

8. Vorrichtung (100) nach einem der Ansprüche 1 bis 7, wobei die zweiten Sensordaten (181) Sensordaten eines Laser-Doppler-Flussmessungssensors, eines Photoplethysmografiesensors, eines Elektrokardiografiesensors oder eines galvanischen Hautreaktionssensors sind.

9. Vorrichtung (100) nach einem der Ansprüche 1 bis 8, wobei das gehirnphysiologische Modell ein Maschinenlernmodell ist.

10. Vorrichtung nach einem der Ansprüche 1 bis 9, wobei in dem Kalibrierungsmodus:
die Benutzeroberfläche konfiguriert ist zum:
Ausgeben eines zweiten Fragebogens bezüglich des physiologischen und/oder kognitiven Zustands des Benutzers wie durch den Benutzer subjektiv wahrgenommen; und
Empfangen eines zweiten Benutzerfeedbacks zu dem Fragebogen, und
die Verarbeitungsschaltung konfiguriert ist, um das gehirnphysiologische Modell für den Benutzer basierend auf dem zweiten Benutzerfeedback zu dem zweiten Fragebogen weiter zu trainieren.

11. Vorrichtung (100) nach einem der Ansprüche 1 bis 10, wobei, in dem Betriebsmodus, die Benutzerschnittstelle (130) konfiguriert ist, um den zweiten Fragebogen in dem Laufe eines Tages wiederholt auszugeben.

12. Vorrichtung (100) nach einem der Ansprüche 1 bis 11, wobei, falls die ersten Sensordaten (171) durch die Sensorschnittstellenschaltung (110) empfangen werden, während die Vorrichtung (100) in dem Betriebsmodus ist, die Verarbeitungsschaltung (120) in dem Betriebsmodus konfiguriert ist, um das gehirnphysiologische Modell basierend auf den ersten Sensordaten (171) zu trainieren, die empfangen werden, während die Vorrichtung (100) in dem Betriebsmodus ist.

13. Vorrichtung (100) nach einem der Ansprüche 1 bis 12, wobei in dem Betriebsmodus:
die Sensorschnittstellenschaltung (110) ferner konfiguriert ist, um Kontextdaten (191) von mindestens einem Kontextsensor (190) zu empfangen, wobei die Kontextdaten (191) eine Aktivität anzeigen, die durch den Benutzer durchgeführt wird; und
die Verarbeitungsschaltung (120) konfiguriert ist, um die Informationen über die Gehirnressourcen des Benutzers durch Verarbeiten der Kontextdaten (191) zusätzlich zu den zweiten Sensordaten (181) mit dem trainierten gehirnphysiologischen Modell zu bestimmen.

14. Computerimplementiertes Verfahren (300) zum Bereitstellen von Informationen über die Gehirnressourcen eines Benutzers, das Verfahren (300) umfassend:
Empfangen (302), in einem Kalibrierungsmodus, erster Sensordaten von einem Elektroenzephalografiesensor an der Sensorschnittstellenschaltung, wobei die ersten Sensordaten ein Elektroenzephalogramm des Benutzers anzeigen;
Empfangen (304), in dem Kalibrierungsmodus und einem Betriebsmodus, zweiter Sensordaten von einem physiologischen Sensor an der Sensorschnittstellenschaltung, wobei die zweiten Sensordaten eine physiologische Eigenschaft des Benutzers anzeigen;
Trainieren (306), in dem Kalibrierungsmodus, eines gehirnphysiologischen Modells für den Benutzer basierend auf den ersten Sensordaten und den zweiten Sensordaten;
Bestimmen (308), in dem Betriebsmodus, der Informationen über die Gehirnressourcen des Benutzers durch Verarbeiten der zweiten Sensordaten mit dem trainierten gehirnphysiologischen Modell, wobei zum Bestimmen der Informationen über die Gehirnressourcen des Benutzers Daten des Elektroenzephalografiesensors nicht mit dem trainierten gehirnphysiologischen Modell verarbeitet werden;
Ausgeben, in dem Betriebsmodus, über eine Benutzerschnittstelle, eines Fragebogens bezüglich eines physiologischen und/oder kognitiven Zustands des Benutzers wie durch den Benutzer subjektiv wahrgenommen;
Empfangen, in dem Betriebsmodus, über die Benutzerschnittstelle eines Benutzerfeedbacks zu dem Fragebogen; Trainieren, in dem Betriebsmodus, des gehirnphysiologischen Modells basierend auf dem Benutzerfeedback zu dem Fragebogen;
Bestimmen einer Abweichung zwischen dem Benutzerfeedback zu dem Fragebogen und den bestimmten Informationen über die Gehirnressourcen des Benutzers in dem Betriebsmodus; und
falls die Abweichung über einem Schwellenwert liegt, Einleiten eines Neukalibrierungsprozesses für das gehirnphysiologische Modell.

15. Computerprogramm, das einen Programmcode zum Veranlassen aufweist, dass die Vorrichtung nach Anspruch 1 das Verfahren nach Anspruch 14 durchführt,
wenn das Programm auf der Verarbeitungsschaltung der Vorrichtung nach Anspruch 1 ausgeführt wird.

## Revendications

1. Appareil (100) permettant de fournir des informations sur des ressources cérébrales d'un utilisateur, l'appareil (100) comprenant une interface utilisateur (130), au moins une circuiterie d'interface de capteur (110) et une circuiterie de traitement (120) couplée à la circuiterie d'interface de capteur (110),
dans lequel, dans un mode d'étalonnage, la circuiterie d'interface de capteur (110) est configurée pour :
recevoir des premières données de capteur (171) à partir d'un capteur d'électroencéphalographie (170), les premières données de capteur (171) indiquant un électroencéphalogramme de l'utilisateur ; et
recevoir des secondes données de capteur (181) à partir d'un capteur physiologique (180), les secondes données de capteur (181) indiquant une propriété physiologique de l'utilisateur,
dans lequel, dans le mode d'étalonnage, la circuiterie de traitement (120) est configurée pour entraîner un modèle cérébral-physiologique pour l'utilisateur en fonction des premières données de capteur (171) et des secondes données de capteur (181),
dans lequel la circuiterie d'interface de capteur (110) est en outre configurée pour recevoir les secondes données de capteur (181) dans un mode de fonctionnement,
dans lequel, dans le mode de fonctionnement, la circuiterie de traitement (120) est configurée pour déterminer les informations concernant les ressources cérébrales de l'utilisateur en traitant les secondes données de capteur (181) avec le modèle cérébral physiologique entraîné,
dans lequel, pour déterminer les informations concernant les ressources cérébrales de l'utilisateur, la circuiterie de traitement (120) est configurée pour ne pas traiter les données de capteur d'électroencéphalographie avec le modèle cérébral-physiologique entraîné,
**caractérisé en ce que** dans le mode de fonctionnement :
l'interface utilisateur (130) est en outre configurée pour :
sortir un questionnaire concernant un état physiologique et/ou cognitif de l'utilisateur, tel qu'il est subjectivement perçu par l'utilisateur ; et
recevoir une réponse de l'utilisateur au questionnaire, et
la circuiterie de traitement (120) est configurée en outre pour :
entraîner le modèle cérébral-physiologique en fonction de la réponse de l'utilisateur au questionnaire ;
déterminer une divergence entre la réponse de l'utilisateur au questionnaire et les informations déterminées sur les ressources cérébrales de l'utilisateur ; et
si la divergence est supérieure à un seuil, lancer un processus de réétalonnage du modèle cérébral-physiologique.

2. Appareil (100) selon la revendication 1, dans lequel, pour l'entraînement du modèle cérébral-physiologique, la circuiterie de traitement (120) est configurée pour personnaliser un modèle cérébral-physiologique brut en fonction des premières données de capteur (171).

3. Appareil (100) selon la revendication 2, dans lequel, pour personnaliser le modèle cérébral-physiologique brut, la circuiterie de traitement (120) est configurée pour déterminer, en fonction des premières données de capteur (171), une énergie cérébrale disponible maximale de l'utilisateur et des caractéristiques d'augmentation et de diminution de l'énergie cérébrale spécifiques d'utilisateur.

4. Appareil (100) selon la revendication 3, dans lequel, pour entraîner le modèle cérébral-physiologique, la circuiterie de traitement (120) est configurée pour :
déterminer une relation entre les premières données de capteur (171) et les secondes données de capteur (181) ; et
déterminer une relation entre les secondes données de capteur (181) et les caractéristiques d'augmentation et de diminution de l'énergie cérébrale spécifiques en fonction de la relation entre les premières données de capteur (171) et les secondes données de capteur (181).

5. Appareil (100) selon l'une quelconque des revendications 1 à 4, dans lequel, dans le mode d'étalonnage, la circuiterie d'interface de capteur (110) est en outre configurée pour recevoir des données contextuelles (191) à partir d'au moins un capteur contextuel (190), les données contextuelles (191) indiquant une activité effectuée par l'utilisateur, et dans lequel la circuiterie de traitement (120) est configurée pour entraîner le modèle cérébral-physiologique en outre en fonction des données contextuelles (191).

6. Appareil (100) selon la revendication 5, dans lequel l'au moins un capteur contextuel (190) est un capteur d'accélération, et dans lequel les données contextuelles (191) indiquent un mouvement respectif d'une ou plusieurs parties du corps de l'utilisateur.

7. Appareil (100) selon la revendication 5 ou la revendication 6, dans lequel l'au moins un capteur contextuel (190) est un capteur de position, et dans lequel les données contextuelles (191) indiquent une géolocalisation de l'utilisateur.

8. Appareil (100) selon l'une quelconque des revendications 1 à 7, dans lequel les secondes données de capteur (181) sont des données de capteur d'un capteur de débitmètre laser Doppler, d'un capteur de photopléthysmographie, d'un capteur d'électrocardiographie ou d'un capteur de réponse galvanique de la peau.

9. Appareil (100) selon l'une quelconque des revendications 1 à 8, dans lequel le modèle cérébral-physiologique est un modèle d'apprentissage automatique.

10. Appareil selon l'une quelconque des revendications 1 à 9, dans lequel, dans le mode d'étalonnage :
l'interface utilisateur est configurée pour :
sortir un second questionnaire concernant l'état physiologique et/ou cognitif de l'utilisateur tel qu'il est subjectivement perçu par l'utilisateur ; et
recevoir une seconde réponse de l'utilisateur au questionnaire, et
la circuiterie de traitement est configurée pour entraîner le modèle cérébral-physiologique pour l'utilisateur en fonction en outre de la seconde réponse de l'utilisateur au second questionnaire.

11. Appareil (100) selon l'une quelconque des revendications 1 à 10, dans lequel, dans le mode de fonctionnement, l'interface utilisateur (130) est configurée pour sortir le second questionnaire de manière répétée tout au long d'une journée.

12. Appareil (100) selon l'une quelconque des revendications 1 à 11, dans lequel, si les premières données de capteur (171) sont reçues par la circuiterie d'interface de capteur (110) lorsque l'appareil (100) est dans le mode de fonctionnement, la circuiterie de traitement (120) est, dans le mode de fonctionnement, configurée pour entraîner le modèle cérébral-physiologique en fonction des premières données de capteur (171) reçues lorsque l'appareil (100) est dans le mode de fonctionnement.

13. Appareil (100) selon l'une quelconque des revendications 1 à 12, dans lequel, dans le mode de fonctionnement :
la circuiterie d'interface de capteur (110) est en outre configurée pour recevoir des données contextuelles (191) à partir d'au moins un capteur contextuel (190), les données contextuelles (191) indiquant une activité effectuée par l'utilisateur ; et
la circuiterie de traitement (120) est configurée pour déterminer les informations sur les ressources cérébrales de l'utilisateur en traitant les données contextuelles (191) en plus des secondes données de capteur (181) avec le modèle cérébral-physiologique entraîné.

14. Procédé (300) mis en oeuvre par ordinateur permettant de fournir des informations sur des ressources cérébrales d'un utilisateur, le procédé (300) comprenant :
la réception (302), dans un mode d'étalonnage, des premières données à partir d'un capteur d'électroencéphalographie au niveau de la circuiterie d'interface de capteur, les premières données de capteur indiquant un électroencéphalogramme de l'utilisateur ;
la réception (304), dans le mode d'étalonnage et dans un mode de fonctionnement, des secondes données de capteur à partir d'un capteur physiologique au niveau de la circuiterie d'interface de capteur, les secondes données de capteur indiquant une propriété physiologique de l'utilisateur ;
l'entraînement (306), dans le mode d'étalonnage, d'un modèle cérébral-physiologique pour l'utilisateur en fonction des premières données de capteur et des secondes données de capteur ;
la détermination (308), dans le mode de fonctionnement, des informations sur les ressources cérébrales de l'utilisateur en traitant les secondes données de capteur avec le modèle cérébral physiologique entraîné, dans lequel, pour déterminer les informations sur les ressources cérébrales de l'utilisateur, des données du capteur d'électroencéphalographie ne sont pas traitées avec le modèle cérébral physiologique entraîné ;
la sortie, dans le mode de fonctionnement, par l'intermédiaire d'une interface utilisateur, d'un questionnaire concernant un état physiologique et/ou cognitif de l'utilisateur tel qu'il est subjectivement perçu par l'utilisateur ;
la réception, dans le mode de fonctionnement, par l'intermédiaire de l'interface utilisateur, d'une réponse de l'utilisateur au questionnaire ; l'entraînement, dans le mode de fonctionnement, du modèle cérébral-physiologique en fonction de la réponse de l'utilisateur au questionnaire ;
la détermination, dans le mode de fonctionnement, d'une divergence entre la réponse de l'utilisateur au questionnaire et les informations déterminées sur les ressources cérébrales de l'utilisateur ; et
si la divergence est supérieure à un seuil, le lancement d'un processus de réétalonnage du modèle cérébral-physiologique.

15. Programme informatique ayant un code de programme permettant d'amener l'appareil de la revendication 1 à effectuer le procédé selon la revendication 14,
lorsque le programme est exécuté sur la circuiterie de traitement de l'appareil de la revendication 1.
